# EUROPEAN PATENT APPLICATION

(11) **EP 0 646 573 A1**
(43) Date of publication of application: **05.04.1995**
(21) Application number: 93115798.6
(22) Date of filing: 30.09.1993
(51) Int. Cl.: C07C 277/08, C07C 279/14

(54) **Preparation of protected and unprotected guanidino-substituted carboxylic acids**

(71) Applicant: HOECHST AKTIENGESELLSCHAFT, D-65929 Frankfurt am Main (DE)
(72) Inventor: Lal, Bansi, Dr., Mulund (W), Bombay 400080 (IN); Gangopadhyay, Ashok Kumar, Mulund (W), Bombay 400080 (IN)

(57) **Abstract**

A process for the preparation of a compound of formula II 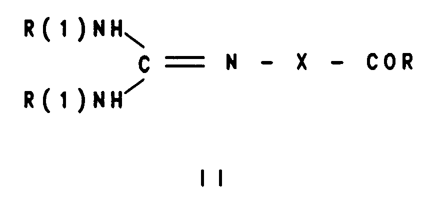
in which are
- X: C₁-C₆-alkyl, C₃-C₆-alkenyl, C₃-C₆-alkynyl, C₃-C₇-cycloalkyl, cycloalkylalkyl, aralkyl, heterocycle or heteroalkyl group;
- R(1): hydrogen, carbobenzyloxy or tert-butyloxycarbonyl group,
- R: OH,
is described which comprises reacting a compound of formula I

H₂N-X-COR I

with trimethylsilyl chloride in an organic solvent,
then reacting with an organic base
and thereafter reacting with a compound of formula III 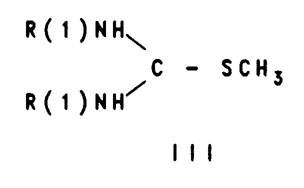
to give compound II.

## Description

The invention relates to the preparation of compounds of formula II, R is OH, from compounds of formula I, R is OH. The method disclosed in the present invention is novel and efficient. 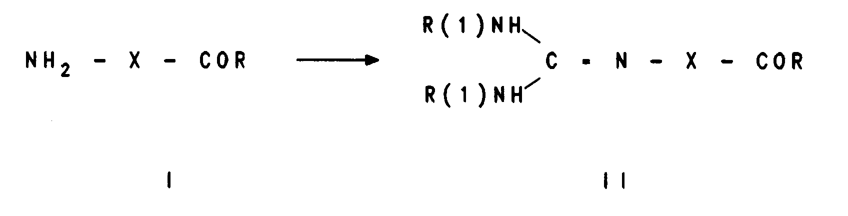
Compounds of formula II are used as intermediates in the synthesis of various biologically active molecules.

### Prior Art:

The preparation of compounds of formula II from compounds of formula I, wherein R is always other than OH is known. For example, compounds of formula I wherein R is OMe or NH₂ when treated with compounds of formula III give compounds of formula II. However, the procedure described in the above prior art does not work with compound of formula I when R is OH.

### Present Invention:

As disclosed above the transformation of compounds of formula I where in R is OH, to compounds of formula II is not successful.

Therefore, it is necessary to protect the carboxylic acid group of formula I wherein R is OH. Moreover, after the reaction, compounds of formula II wherein the carboxylic group is protected have to be deprotected. This involves additional steps. The present invention describes a direct method wherein separate protection and deprotection step are avoided.

### Detailed description of the invention

The present invention describes a process for the preparation of compounds of the formula II 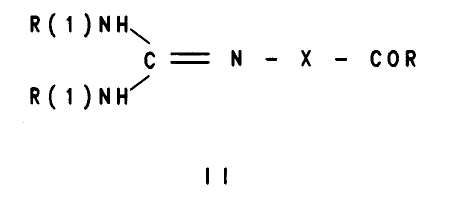
wherein
X stands for C₁-C₆-alkyl, C₃-C₆-alkenyl, C₃-C₆-alkynyl, C₃-C₇-cycloalkyl, cycloalkylalkyl, aralkyl, heterocycle or heteroalkyl group;
R(1) stands for hydrogen, carbobenzyloxy or tert-butyloxycarbonyl group.

The term C₁-C₆-alkyl relates to straight-chain or branched chain saturated hydrocarbon radicals such as methyl, ethyl, propyl, t-butyl, pentyl, hexyl which are unsubstituted or substituted by a protected amino, hydroxy or a thiol group.

The term C₃-C₆-alkenyl stands for straight or branched chain C₃-C₆-hydrocarbon radicals containing at least one unsaturation such as vinyl.

The term alkynyl stands for straight or branched chain hydrocarbon radicals containing 3 to 6 carbons and containing at least one acetylenic bond.

The term C₃-C₇-cycloalkyl stands for saturated or unsaturated cyclic compounds, which may further be substituted by functional groups such as, C₁-C₃-alkyl, C₁-C₃-alkoxy, halogen.

The term aryl stands for an aromatic nucleus preferably a phenyl group which is unsubstituted or substituted by 1, 2 or 3 identical or different substituents such as halogen, C₁-C₃-alkyl, C₁-C₃-alkoxy, C₃-C₆-alkenyl, nitro or heterocycle.

The term aralkyl refers to a group such as a benzyl group.

The term heterocycle stands for a cyclic structure containing one or more heteroatoms such as N, O, S represented by rings such as piperidine, morpholine, piperazine, pyrrolidine.

The term heteroaryl stands for an aromatic ring containing one or more heteroatoms in the ring such as pyridine.

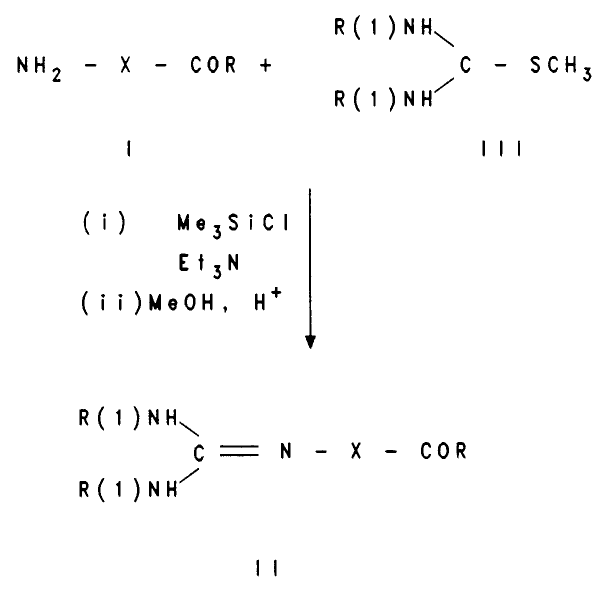

The novel process described herein has tremendous utility in the preparation of suitably protected amino acids which find vast application in the synthesis of biologically active peptides.

The process comprises of first treating compounds of formula I in which R is OH wherein X has the same meaning as defined above with trimethyl silylchloride using an organic solvent such as methylene chloride or acetonitrile or DMF or THF or dioxane or chloroform initially at room temperature and then at reflux temperature of the solvent used for two hours. The reaction mixture after cooling is further treated with an organic base such as triethylamine or dimethylaminopyridine followed by the addition of a compound of the formula III wherein R(1) has the same meaning as defined above at room temperature. The reaction mixture is then refluxed for one to sixteen hours, cooled again to room temperature and diluted with methanol under stirring.

To isolate the compound, the reaction mixture is concentrated under vacuum, the residue obtained is diluted with water and the aqueous solution acidified with citric acid to pH 2. The precipitated solid is extracted with organic solvent such as ethyl acetate and the organic layer after separation is washed with brine, dried over anhydrous Na₂SO₄ and evaporated to dryness and the residual solid is purified by recrystallisation.

The following examples illustrate the invention but do not limit the scope of the invention.

### Example 1

### N,N'-Dibenzyloxycarbonylguanidinoacetic acid

Trimethylsilyl chloride (5.08 ml; 40 mmol) was added dropwise at room temperature over a period of 5 minutes to a well stirred suspension of finely powdered glycine (3.0 g; mmole) in dichloromethane (50 ml). The reaction mixture was then refluxed for two hours. After cooling to room temperature, triethylamine (5.6 ml, 40 mmole) was added followed by a solution of dicarbobenzyloxy-S-methyl isothiourea (8.95 g; 25 m. mole) in dichloromethane (30 ml). The reaction mixture was then refluxed for sixteen hours. The reaction mixture after cooling to room temperature was diluted with methanol (25 ml) under stirring and then concentrated under vacuum. The residue obtained was diluted with water, acidified with citric acid to pH 2 and extracted with ethyl acetate. The ethyl acetate layer after separation was washed with brine, dried over anhydrous sodium sulphate, filtered and concentrated. The residue obtained was crystallized from ethyl acetate/petrol ether to obtain pure product 9.4 g
(yield 97.5 %), m.p. 135°-137°C.

### Example 2

### 3-(N,N'-Dibenzyloxycarbonylguanidino)propionic acid

Following the procedure described in Example 1 using 3-amino propionic acid in place of glycine, the title compound was obtained in 92 % yield, m.p. 152°-154°C (EtOAc/Petrol ether).

### Example 3

### 4-(N-N'-Dibenzyloxycarbonylguanidino)butyric acid

Following the procedure described in Example 1 using 4-amino butyric acid in place of glycine, the title compound was obtained in 90 % yield, m.p. 139°-140°C.

### Example 4

### 5-(N,N'-Dibenzyloxycarbonylguanidino)valeric acid

Following the procedure described in Example 1, using 5-amino valeric acid in place of glycine, the title compound was obtained in 90.6 % yield, m.p. 115°C (EtOA/Petrol ether).

### Example 5

### 6-(N,N'-Dibenzyloxycarbonylguanidino)caproic acid

Following the procedure described in Example 1, using 6-amino caproic acid in place of glycine, the title compound was obtained in 80 % yield, m.p. 103°C-104°C (EtOAc/Petrol ether).

### Example 6

### 2-(N,N'-Dibenzyloxycarbonylguanidino)-3-phenylpropionic acid

Following the procedure described in Example 1 using phenylalanine in place of glycine, the title product was obtained in 84.3 % yield, m.p. 135°-136°C (EtoAc/Pet. ether).

### Example 7

Following the procedure described in Example 1 using glycylglycine in place of glycine, the title compound was obtained in yield 68.5 %, m.p. 90°-92°C.

### Example 8

### 4-(N,N'-Dibenzyloxycarbonylguanidino)butyric acid

Triethylamine (2.8 ml; 20 mmole) and trimethylsilyl chloride (2.54 ml; 20 mmole) were added successively to a well stirred suspension of 4-aminobutyric acid hydrochloride (2.8 g, 20 mmole) in dichloromethane (30 ml) at room temperature. After the addition was over, the reaction mixture refluxed for 2 hours, cooled to room temperature and triethylamine (2.8 ml; 20 mmole) was added slowly under stirring followed by dicarbobenzyloxy-5-methyl isothiourea (3.58 g, 10 mmole) in methylenedichloride (20 ml). The reaction mixture was refluxed for 16 hours. It was brought to room temperature and excess methanol (10 ml) was added with stirring. The reaction mixture then concentrated under vacuum and the residue was taken up in water, acidified with citric acid. The precipitated solid was extracted with ethyl acetate. The extract was washed with brine dried over anhydrous sodium sulphate, filtered and the filtrate evaporated to dryness. The residue obtained was crystallized from ethyl acetate/Petrol ether to obtain 3.2 g of the title compound (yield 90 %), m.p. 139°-140°C.

## Claims

1. Process for the preparation of a compound of formula II 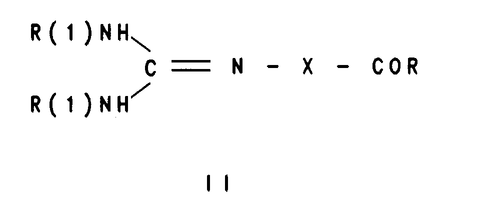 in which are
X C₁-C₆-alkyl, C₃-C₆-alkenyl, C₃-C₆-alkynyl, C₃-C₇-cycloalkyl, cycloalkylalkyl, aralkyl, heterocycle or heteroalkyl group;
R(1) hydrogen, carbobenzyloxy or tert-butyloxycarbonyl group,
R OH,
which comprises
reacting a compound of formula I
H₂N-X-COR I
with trimethylsilyl chloride in an organic solvent,
then reacting with an organic base
and thereafter reacting with a compound of formula III 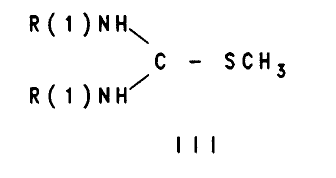 to give compound II.
